# FASCICULE DE BREVET EUROPEEN

(11) **EP 0 753 289 B1**
(45) Date de publication et mention de la délivrance du brevet: **26.11.2003**
(21) Numéro de dépôt: 96420226.1
(22) Date de dépôt: 02.07.1996
(51) Int. Cl.: A61F 2/04, A61M 25/00

(54) **Cathéter fractionnable**
Teilbarer Katheter
Fractionable catheter

(30) Priorité: 10.07.1995 FR 9508682
(43) Date de publication de la demande: 15.01.1997
(73) Titulaire: Devonec, Marian, F-01700 Miribel (FR)
(72) Inventeur: Devonec, Marian, 01700 Miribel (FR); Sherwood, Les, Bloomington, Indiana 47404 (US); Desmond, Joe, Bloomington, Indiana 47403 (US)
(74) Mandataire: Guerre, Dominique

(56) Documents cités:
- EP-A- 0 341 988
- WO-A-94/18907

## Description

La présente invention concerne un cathéter fractionnable, destiné à être utilisé dans le traitement des obstacles prostatiques chez l'homme.

Les solutions techniques proposées pour traiter les obstacles prostatiques sont bien connues dans l'état de la technique. De manière générale, ces solutions impliquent le placement d'un cathéter intra-urétral (en anglais, IUC), dans lequel un passage libre est créé entre la vessie et l'extérieur du corps humain, de façon à assurer l'écoulement permanent de l'urine au dehors. Un tel cathéter est le cathéter urétral dit de Foley ou sonde à demeure.

Ce cathéter consiste en un tube en une seule pièce (monobloc), qui comprend une lumière principale pour drainer l'urine et une lumière de petit diamètre dans la paroi du tube, cette dernière permettant de gonfler un ballonnet situé à proximité de l'extrémité distale du tube.

L'extrémité distale est souple et comporte un orifice qui communique avec la lumière principale, assurant ainsi le drainage permanent de l'urine à partir de la vessie, et un ballonnet situé légèrement en retrait ou en dessous par rapport à l'orifice de drainage urinaire. Une fois gonflé, le ballonnet sert à maintenir en permanence l'orifice de drainage à l'intérieur de la vessie et à retenir le cathéter dans le canal urétral.

L'extrémité proximale du cathéter comporte une ouverture pour le drainage de l'urine, qui est en continuité d'écoulement avec la lumière principale du tube, et présente une forme conique permettant son raccordement à un collecteur d'urine, ainsi qu'une valve d'injection-aspiration en continuité avec la lumière de petit diamètre dans la paroi, qui permet de gonf ler et dégonfler le ballonnet.

Ce type de cathéter assure un écoulement permanent de l'urine à l'intérieur du cathéter. Il est souple pour s'adapter aux différentes courbures et angulations de l'urètre. Mais sa paroi est suffisamment rigide pour empêcher tout écrasement ou plicature du cathéter, qui pourrait en particulier obstruer la lumière principale et gêner l'écoulement de l'urine. Les avantages de ce système sont sa mise en place et retrait simples, qui ne nécessitent pas d'anesthésie.

Toutefois, un tel cathéter présente de nombreux inconvénients, car il est inconfortable et responsable de complications. Cet inconfort est dû notamment à :
- l'intubation de l'urètre sur toute sa longueur (du col de la vessie jusqu'au méat urétral) par un corps étranger, alors que l'obstacle est limité au segment prostatique ;
- la connexion du cathéter à un système collecteur d'urine qu'il faut vider plusieurs fois par jour ;
- l'écoulement permanent de sécrétions urétrales le long du cathéter, nécessitant des soins d'hygiène locale pluri-quotidiens ;
- l'impossibilité d'une activité sexuelle ;
- et les fuites possibles d'urine.

Parmi les complications associées à l'utilisation de tels cathéters, les plus courantes sont :
- l'infection urinaire, par exemple des cystites ;
- la sténose de l'urètre, plus particulièrement au niveau de la jonction péno-scrotale, la région rétroméatique ou le méat urétral ;
- l'incrustation du ballonnet par les sels présents dans l'urine, cette incrustation pouvant favoriser l'infection urinaire et la formation de calculs, et gêner le dégonflage et le retrait du ballonnet, qui devient alors traumatique pour le patient ; il est par conséquent nécessaire de changer le cathéter toutes les six semaines.

La réussite de l'utilisation d'un tel système dépend aussi bien de la manipulation sensible et correcte du médecin, que de l'anatomie individuelle de chaque malade.

La présente invention propose alors un cathéter amélioré qui pallie les inconvénients associés au système connu et décrit ci-dessus.

Plus particulièrement, la présente invention a pour objet un cathéter dont la mise en place dans l'urètre peut être réalisée sans l'aide d'un moyen d'imagerie particulier, et demeure relativement indépendante de l'anatomie du patient à traiter et de l'habileté du médecin.

WO-A-9 418 907 décrit un cathéter urétral fractionnable, qui comprend un moyen distal de cathétérisation et un moyen d'introduction, assemblés l'un par rapport à l'autre de manière alignée, pour l'introduction du cathéter dans l'urètre, et dissociables l'un par rapport à l'autre, pour laisser en place le moyen de cathétérisation dans l'urètre. Le cathéter selon la présente invention présente en combinaison les caractéristiques suivantes :
- le moyen de cathétérisation est relativement flexible de manière à se conformer à l'urètre de la région prostatique, mais suffisamment rigide de façon à maintenir un passage artificiel dans cette dernière, et comprend deux éléments tubulaires, dits respectivement supérieur et inférieur, destinés à être placés respectivement dans la partie supérieure et la partie inférieure du canal urétral, de part et d'autre du sphincter strié, lesdits éléments tubulaires étant reliés l'un à l'autre par un moyen de liaison flexible et déformable, se logeant dans l'orifice du sphincter strié ;
- le moyen d'introduction comprend un tube externe poussoir, et une tige interne de section extérieure adaptée à la section intérieure du tube externe , ledit moyen d'introduction ayant une longueur adaptée pour intuber le moyen de cathétérisation jusqu'à l'extrémité distale de l'élément supérieur ;
- en combinaison, le moyen d'introduction comprend un moyen distal de positionnement provisoire, par rapport au côté inférieur du sphincter strié, et d'autre part, le moyen d'introduction comprend un moyen extérieur d'activation du moyen de positionnement provisoire, disposé à une extrémité proximale du moyen d'introduction ;
et premièrement le tube externe poussoir a un diamètre extérieur moins important que le diamètre intérieur des deux éléments tubulaires du moyen de cathétérisation, de façon à permettre un déplacement longitudinal avec jeu fonctionnel du moyen de cathétérisation sur le moyen d'introduction, deuxièmement le tube externe poussoir a une longueur sensiblement égale à celle de la tige interne, et troisièmement le moyen de cathétérisation est disposé sur le moyen d'introduction, en sorte que le moyen distal de positionnement provisoire se situe entre les deux éléments tubulaires du moyen de cathétérisation.

Par l'expression "provisoire", on doit comprendre que le moyen de positionnement ne fonctionne pas de façon à maintenir le moyen de cathérisation en place définitivement pendant le traitement, c'est-à-dire de manière passive, mais plutôt qu'il peut être activé, uniquement lors de l'introduction du cathéter, afin d'apporter une aide positive au médecin, après quoi, il peut être désactivé, afin de laisser le moyen de cathétérisation en bonne position par rapport au sphincter strié.

Le positionnement du cathéter fractionnable selon la présente invention s'effectue par la coopération des moyens définis ci-dessus, par rapport au sphincter strié, et non par rapport au col de la vessie, ce qui permet également de le positionner de manière reproductible, avec uniquement la sensibilité du médecin, sans être obligé à recourir à une aide instrumentale quelconque de visualisation ou de détection de position.

Par ailleurs, en prévoyant un élément tubulaire supérieur du moyen de cathétérisation suffisamment long par rapport au sphincter strié, il devient possible d'établir une communication avec la vessie, quelles que soient la taille et/ou la conformation de la partie prostatique de l'urètre, et par conséquent de prévoir une taille unique de cathéter.

Le moyen de positionnement provisoire peut être constitué par un ballon gonflable, circonscrivant par exemple l'élément tubulaire inférieur, et des moyens de communication relient, dans la position assemblée du cathéter, d'un côté le ballon, et de l'autre côté le moyen extérieur d'activation, à savoir une source extérieure de fluide.

Le moyen de positionnement provisoire peut comprendre une pluralité d'éléments d'appui distribués autour du tube externe poussoir, agencés chacun pour prendre deux positions, à savoir une position rétractée radialement, et une position déployée saillant radialement à partir du tube externe poussoir. Et le moyen extérieur d'activation est un moyen mécanique pour agir à distance sur les éléments d'appui, et les faire passer de la position déployée à la position rétractée, et inversement.

La présente invention sera maintenant décrite plus en détail, en se référant au dessin annexé, représentant un mode d'exécution préféré, dans lequel :
- la Figure 1 représente une vue schématique, de face, d'un cathéter fractionnable ;
- la Figure 2 représente une vue en coupe longitudinale, à échelle agrandie, du cathéter fractionnable de la Figure 1 ;
- la Figure 3 représente schématiquement une vue en coupe longitudinale de la région prostatique masculine et le cathéter de la Figure 1 avant son introduction ;
- la Figure 4 montre la même vue en coupe que la Figure 3, après une première étape d'introduction du cathéter selon Figure 1 jusqu'au sphincter strié ;
- la Figure 5 représente l'étape suivante, dans laquelle le moyen de positionnement provisoire du cathéter selon Figure 1 est activé dans la région bulbaire de l'urètre ;
- la Figure 6 représente l'étape de positionnement, à l'aide du moyen de positionnement provisoire, du moyen de cathétérisation selon Figure 1 ;
- la Figure 7 montre la mise en place définitive du moyen de cathétérisation selon Figure 1 ;
- la Figure 8 représente la fermeture du sphincter autour des moyens de liaison flexible reliant les éléments tubulaires supérieur et inférieur du moyen de cathétérisation selon Figure 1 ;
- la Figure 9 représente le cathéter selon Figure 1, après désactivation du moyen de positionnement provisoire, et séparation du moyen d'introduction par rapport au moyen de cathétérisation ;
- la Figure 10 représente la position finale du moyen distal de cathétérisation selon Figure 1, après retrait total du moyen proximal d'introduction, montrant le passage libre d'écoulement entre la vessie et l'urètre bulbaire, sous le contrôle du sphincter.
- la Figure 11 représente une vue schématique, de face, d'un cathéter fractionnable, dans la position rétractée des éléments d'appui du moyen distal de positionnement provisoire ;
- la Figure 12 représente le cathéter selon Figure 11, avec vue en coupe partielle, dans la position déployée des éléments d'appui du moyen distal de positionnement provisoire.

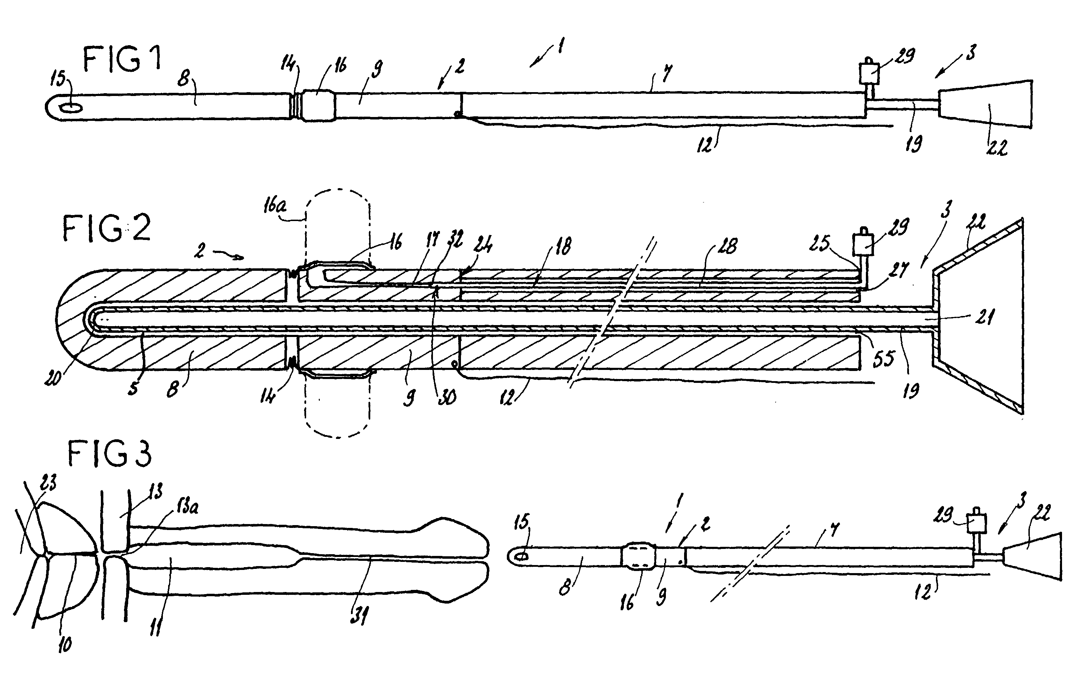

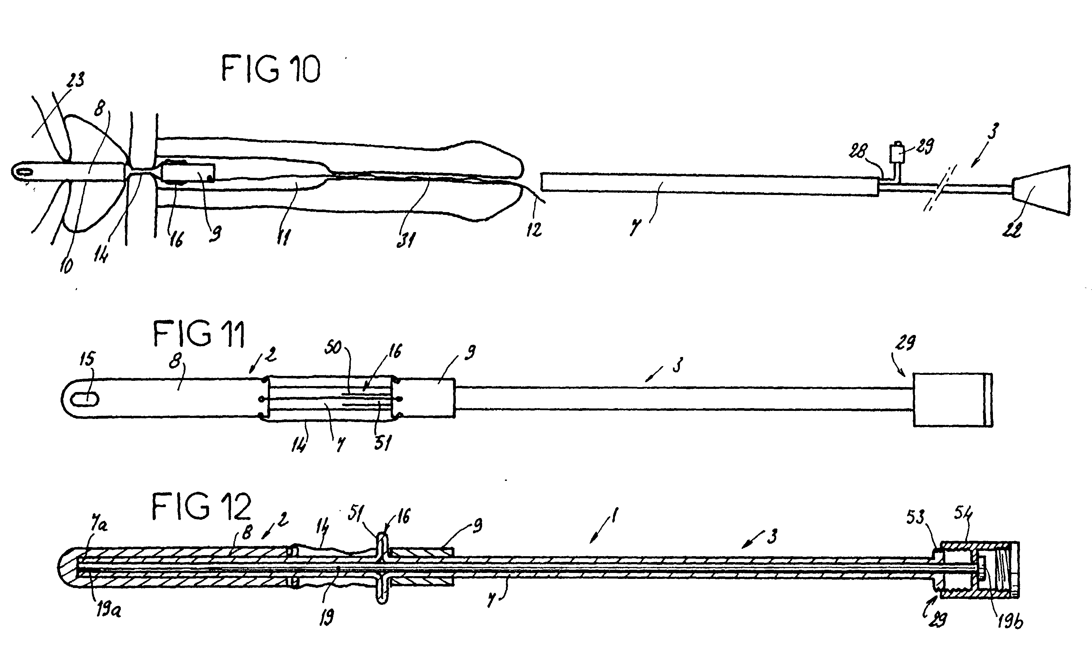

Comme représenté schématiquement aux Figures 1 et 2, un cathéter fractionnable est désigné de façon générale par le numéro de référence 1. Ce cathéter 1 comprend un moyen distal de cathétérisation 2 et un moyen proximal d'introduction 3. Comme montré en particulier par la comparaison entre les figures 3 et 10, le moyen distal de cathétérisation 2 est assemblé au départ, par rapport au moyen d'introduction 3, de manière alignée ou coaxiale, pour l'introduction du cathéter dans l'urètre, et finalement, après mise en place du moyen de cathétérisation 2, le moyen d'introduction 3 est dissocié et extrait de l'urètre.

Le moyen de cathétérisation 2 est relativement flexible, de façon à se conformer aux dimensions et forme naturelles de l'urètre de la région prostatique, mais suffisamment rigide de façon à maintenir un passage artificiel dans cette dernière. Le moyen 2 comporte au moins une lumière 5 longitudinale, le traversant d'une extrémité à l'autre, et il est destiné et adapté à être placé et maintenu dans la région prostatique.

Le moyen de cathétérisation 2 comprend deux éléments tubulaires, respectivement supérieur 8 et inférieur 9, destinés à être placés dans la partie supérieure 10 et la partie inférieure 11 du canal urétral, de part et d'autre du sphincter strié 13. Les éléments tubulaires 8 et 9 présentent la même section interne, avec le même diamètre intérieur. Les éléments tubulaires supérieur 8 et inférieur 9 sont reliés l'un à l'autre par un moyen de liaison 14 flexible et déformable, destiné à être placé dans l'orifice 13a du sphincter 13. Le moyen de liaison 14 permet le jeu normal du sphincter strié 13 et assure l'écoulement du liquide entre les éléments tubulaires supérieur et inférieur 8, 9. Ce moyen est constitué par des fils en polyamide, une membrane tubulaire ou un tube en matière flexible, par exemple du polyéthylène ou polypropylène.

L'élément tubulaire supérieur 8 présente, à sa partie distale, un ou deux orifices 15 de drainage traversant sa paroi extérieure, et communiquant avec la lumière longitudinale 5. Son extrémité distale est de préférence souple, par exemple mousse synthétique, et son extrémité proximale communique avec le moyen de liaison 14.

L'élément tubulaire inférieur 9 présente, en plus de la lumière 5, un ballon ou ballonnet 16 gonflable le circonscrivant, en toute matière appropriée, par exemple en caoutchouc, disposé du côté distal, près et en dessous du moyen de liaison 14, par exemple à 2 mm de celui-ci. Le ballonnet 16, qui est situé près et en dessous du moyen de liaison 14, a une longueur préférentielle d'environ 15 à 20 mm. Le ballonnet peut être fixé sur ou intégré dans l'élément tubulaire inférieur 9. La Figure 2 montre la forme du ballon 16 à l'état désactivé, c'est-à-dire dégonflé, son état activé 16a, c'est-à-dire gonflé, étant représenté en traits mixtes. De préférence, le ballonnet 16 est intégré au dit élément tubulaire 9, et peut être gonflé par l'intermédiaire d'un passage 17 supplémentaire longitudinal de connexion, ménagé dans la paroi de cet élément tubulaire 9, qui correspond avec une lumière 27 longitudinale de diamètre légèrement supérieur, ménagée dans le tube poussoir 7, et avec laquelle il est en alignement. La section transversale du passage de connexion 17 est plus petite que celle de la lumière 27 dans le tube poussoir 7. Le passage de connexion 17 communique avec le ballon ou ballonnet 16 par un orifice situé près de l'extrémité distale de l'élément tubulaire inférieur 9. Avantageusement, l'élément tubulaire inférieur 9 peut également comporter un fil de retrait 12, fixé dans une zone proximale dudit élément 9, et destiné à faciliter le retrait du moyen de cathétérisation 2 en cas de besoin.

Le moyen d'introduction 3 comprend d'une part, un tube externe poussoir 7 de diamètre extérieur voisin de celui des deux éléments tubulaires 8, 9 du moyen de cathétérisation 2, et d'autre part, une tige interne d'alignement 19, semi-rigide, de section extérieure adaptée à la section intérieure du tube 7 et du moyen de cathétérisation 2, c'est-à-dire des deux éléments tubulaires 8 et 9, et de longueur adaptée pour intuber à la fois le tube poussoir 7 et le moyen de cathétérisation 2. Le tube poussoir 7 a donc une longueur inférieure à celle de la tige d'alignement 19. Le tube poussoir 7 du moyen d'introduction 3 présente une lumière principale 55 qui communique selon la même section avec la lumière 5 ménagée au sein du moyen de cathétérisation. Ce tube 7 comporte également des moyens de communication 18 pour établir une connexion, d'une part, à son extrémité distale 24, avec les moyens de positionnement provisoire 16 de l'élément tubulaire inférieur 9, et d'autre part à son extrémité proximale 25 opposée, avec un moyen 29 d'activation extérieure, pour activer lesdits moyens de positionnement provisoire 16 au moyen d'une source extérieure de fluide. De préférence, et comme représenté à la figure 2, les moyens de communication 18 servent au passage du fluide de gonflage, et sont constitués par la lumière longitudinale 27, et un tube métallique creux 28, par exemple une canule souple, d'introduction du fluide de gonflage. Le tube creux 28 présente un diamètre extérieur plus important que le diamètre intérieur du passage de connexion 17, de façon à ce que ledit tube 28 s'insère de manière à être étanche aux fluides, par son extrémité distale dans l'extrémité proximale du passage de connexion 17. Ce tube creux 28 présente un diamètre extérieur moins important que celui de la lumière 27 de manière à permettre son déplacement longitudinal, avec jeu fonctionnel à l'intérieur de ladite lumière.

Le tube métallique 28 est muni du moyen d'activation ou commande 29, commandant l'écoulement du fluide de gonflage, à son extrémité proximale ; et son extrémité distale 32 assure la communication avec le ballon 16. Le moyen d'activation 29, comme représenté à la figure 2, constitue par exemple, un robinet à une voie ou une valve d'injection, dont l'axe est incliné par rapport à l'axe longitudinal du tube. Dans le cas où le tube métallique 28 est une canule ou une aiguille, celle-ci traverse toute la longueur du tube 7 et au-delà de son extrémité distale 24, lorsque la valve d'injection 29 est en butée contre l'extrémité proximale 25 du tube 7. Les moyens de connexion étanche 30 avec le ballon 16 sont donc constitués, d'une part, par l'extrémité distale 32 du tube métallique creux 28, et d'autre part, par le passage de connexion 17, prévu dans l'élément tubulaire inférieur 9, et se raccordant de manière étanche sur l'extrémité 32.

La tige d'alignement 19 creuse, semi-rigide, du moyen d'introduction 3, présente une section transversale extérieure adaptée à être logée dans les lumières 5 et 55 des moyens de cathétérisation 2 et d'introduction 3, une extrémité distale 20 adaptée pour communiquer avec l'intérieur de l'élément tubulaire supérieur 8, et une extrémité proximale 21 ouverte sous forme de butée 22. Cette tige assure la cohésion, l'unicité et l'alignement des moyens de cathétérisation 2 et d'introduction 3 avant l'insertion du cathéter, et peut glisser le long des lumières 5 et 55 avec un jeu fonctionnel approprié. A son extrémité distale, la tige est munie d'un oeil ou de deux yeux, non représentés, communiquant avec l'intérieur des lumières 5 et 55, et situés en regard des deux orifices 15 de l'élément tubulaire supérieur 8, dans la position assemblée des moyens de cathétérisation 2 et d'introduction 3. Ceci permet au médecin de constater l'arrivée du cathéter dans la vessie 23, car il y aura alors écoulement d'urine.

Le cathéter fractionnable représenté au figures 11 et 12 se distingue de celui précédemment décrit, par les caractéristiques suivantes.

Le moyen d'introduction 3, et plus précisément le tube externe poussoir 7 comprennent le moyen distal de positionnement provisoire 16.

Le tube externe poussoir 7 a un diamètre extérieur moins important que le diamètre intérieur des deux éléments tubulaires 8 et 9 du moyen de cathétérisation 2, de façon à permettre un déplacement longitudinal avec jeu fonctionnel du moyen de cathétérisation 2 sur ou par rapport au moyen d'introduction 3. Le tube externe poussoir 7 a par ailleurs une longueur sensiblement égale à celle de la tige interne 19. L'extrémité distale 7a du tube externe poussoir 7 vient en butée contre l' extrémité distale interne de l'élément tubulaire supérieur 8 du moyen de cathétérisation 2. Et le moyen de cathétérisation 2 est disposé sur le moyen d'introduction 3, en sorte que le moyen distal 16 de positionnement provisoire se situe entre les deux éléments tubulaires 8 et 9 du moyen de cathétérisation, c'est-à-dire avant la mise en place du cathéter fractionnable. La tige interne 19 a quant à elle une longueur sensiblement égale à celle du tube externe poussoir 7, et est fixée en translation à son extrémité distale 19a, sur l'extrémité distale 7a du tube externe poussoir 7.

Le moyen distal 16 de positionnement provisoire comprend une pluralité d'éléments d'appui 51, distribués autour du tube externe poussoir 7, agencés chacun pour prendre deux positions, à savoir une position rétractée radialement, et une position déployée saillant radialement à partir du tube externe poussoir 7. Pour ce faire, le tube externe 7 comporte localement, c'est à dire à l'endroit du moyen distal 16 de positionnement provisoire, des découpes axiales 50 ménageant entre elles des languettes 51. Chaque languette 51 est pliable radialement entre deux positions, à savoir une première position pliée en son milieu vers l'extérieur (Cf. Figure 12), et une position dépliée (Cf. Figure 11), comprise dans la paroi du tube externe poussoir 7. Ces languettes 51 forment ou constituent ainsi des éléments d'appui contre le côté inférieur du sphincter strié.

Le moyen extérieur 29 d'activation comprend un moyen mécanique pour déplacer axialement le tube externe 7 par rapport à la tige interne 19, et plier et déplier en correspondance les languettes 51 du moyen distal 16 de positionnement provisoire. Ce moyen mécanique comprend un filetage 53, disposé à l'extérieur et à l'extrémité distale du tube externe 7, et une collerette 54, taraudée intérieurement coopérant avec le filetage 53, et en appui en libre rotation contre l'extrémité distale 19b de la tige interne 19.

La mise en place du cathéter selon l'invention sera maintenant décrite, en se référant aux Figures 3 à 10.

La Figure 3 montre le cathéter assemblé, en position prête à l'introduction, ainsi qu'une vue schématisée de l'urètre masculin. Celui-ci comprend, de manière générale, cinq segments de diamètre, longueur et orientation très différents, à savoir :
- un segment pénien 31, de petit diamètre, long de 100 mm environ, et qui est orienté selon la position du pénis ;
- un segment bulbaire 11, de fort diamètre, long de 70 mm environ, en position fixe et horizontale ;
- un segment membraneux 13a, de petit diamètre, long de 10 mm environ, en position verticale ; c'est le segment de traversée du plancher pelvien, et du sphincter strié 13 ;
- et un segment prostatique 10, de diamètre et géométrie variables selon l'anatomie de la prostate, long de 30 à 70 mm, et d'orientation verticale.

Ces différentes orientations ne sont pas représentées dans les figures, mais il est important de retenir que l'urètre bulbaire 11 présente un calibre beaucoup plus important que les autres segments, et que l'angulation entre les segments prostatique 10 et bulbaire 11 est de l'ordre de 120°.

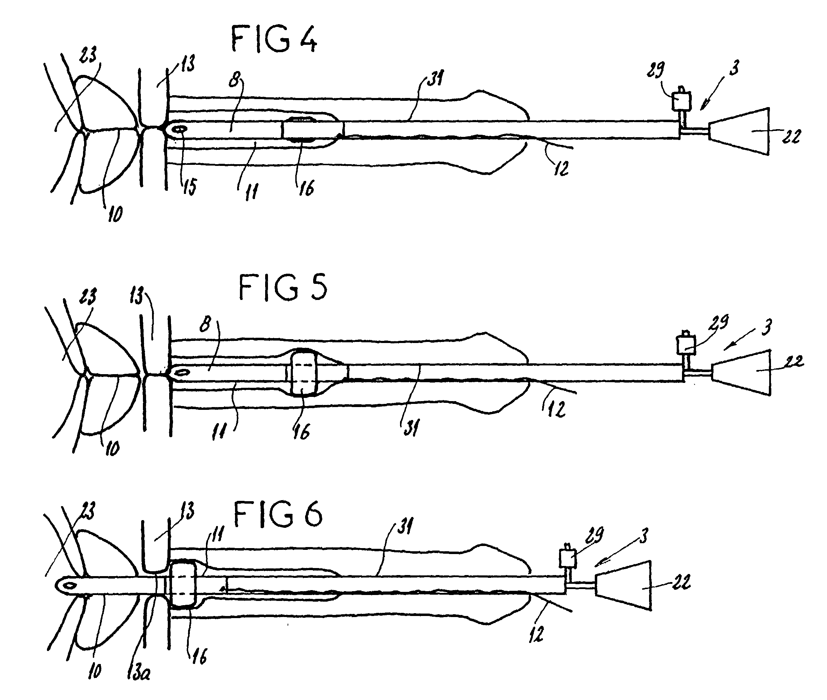

Le cathéter 1 est d'abord introduit dans l'urètre pénien 31 et puis l'urètre bulbaire 11, jusqu' à ce que le médecin sente le contact de son extrémité distale avec le sphincter strié 13 (cf. Figure 4). Celui-ci est fermé en permanence en dehors de la miction, ce qui nécessite de vaincre une certaine résistance pour le franchir.

L'activation du moyen de positionnement provisoire 16 est représentée dans la Figure 5. Selon le premier mode d'exécution, le ballonnet 16 est gonflé par la valve 29 avec une quantité appropriée de fluide, par exemple avec 1,5 à 2 ml d'eau stérile. La paroi du ballonnet 16 vient se plaquer contre la paroi du segment bulbaire 11 de l'urètre dont le diamètre est, à cet endroit, plus important que celui du segment pénien 31. Selon le deuxième mode d'exécution, les languettes 51 sont pliées sensiblement en leur milieu, et viennent contre la paroi du segment bulbaire 11

Dans l'étape suivante (cf. Figure 6), le médecin fait avancer le cathéter jusqu'à ce que le moyen 16, dans le segment bulbaire 11, vienne en butée contre le sphincter et soit arrêté par ce dernier. Ceci permet à l'élément tubulaire supérieur 8 de franchir en grande partie le sphincter strié 13.

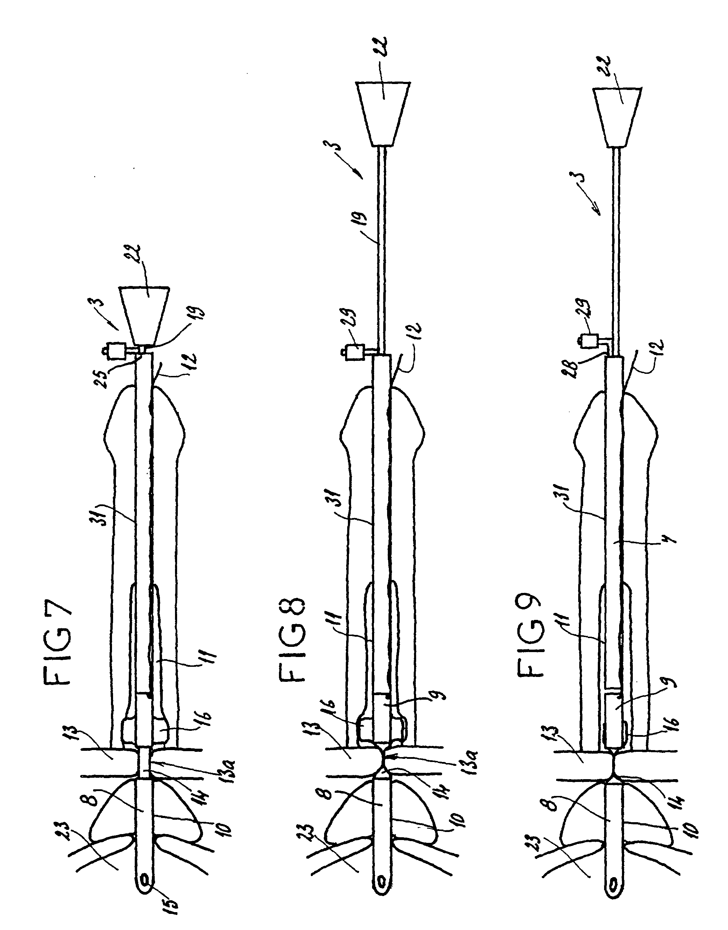

L'étape suivante, représentée par la Figure 7, consiste à tendre les moyens de liaison 14, en l'occurrence des fils de polyamide, en poussant l'élément tubulaire supérieur 8 avec le moyen d'introduction 3, par exemple selon le premier mode d'exécution en poussant l'élément 8 avec la tige interne 19 jusqu'à ce que son cône 22 vienne en butée contre l'extrémité proximale 25 du tube 7. Cette manoeuvre permet audit élément tubulaire supérieur 8 de franchir complètement le sphincter et d'intuber en totalité le segment prostatique 10. L'écoulement de l'urine par l'intérieur de la tige interne 19 garantit que l'extrémité distale de l'élément tubulaire supérieur 8 est arrivée dans la vessie.

La Figure 8 montre le commencement de la séparation ou dissociation du moyen de cathétérisation 2 et du moyen d'introduction 3. En particulier, la tige interne 19 est retirée partiellement, d'une longueur légèrement supérieure à celle du moyen de cathétérisation, une fois en place. A ce moment, le sphincter strié 13 vient se refermer autour du moyen de liaison 14, bloquant ainsi les éléments tubulaires 8 et 9 dans les segments prostatique 10 et bulbaire 11 respectivement.

Ensuite, l'aiguille 28 est retirée d'une longueur supérieure à celle qui intubait le passage supplémentaire 17 de l'élément tubulaire inférieur 9, par exemple, d'environ 7 mm. Le ballonnet se dégonfle alors immédiatement (cf. Figure 9). Ou alors les languettes 51 sont dépliées, et se confondent sensiblement dans la paroi du tube poussoir 7.

La figure 10 montre l'étape finale et le retrait en bloc du moyen d'introduction 3. En effet, le médecin retire à la fois la tige interne 19, et le tube poussoir 7, et l'aiguille 28 (selon le premier mode d'exécution).

Le retrait du moyen de cathétérisation 2 peut s'effectuer séparément, par traction sur l'extrémité proximale de l'élément tubulaire inférieur 9, soit à l'aide du fil 12, préalablement fixé à ce niveau, soit à l'aide d'une petite pince, sous contrôle d'un endoscope.

## Revendications

1. Cathéter fractionnable (1 ) destiné à être utilisé dans le traitement des obstacles prostatiques chez l'homme, comprenant un moyen distal de cathétérisation (2) et un moyen d'introduction (3), assemblés l'un par rapport à l'autre de manière alignée, pour l'introduction du cathéter dans l'urètre, et dissociables l'un par rapport à l'autre, pour laisser en place le moyen de cathétérisation (2) dans l'urètre, cathéter selon lequel :
- le moyen de cathétérisation (2), relativement flexible de manière à se conformer à l'urètre de la région prostatique, mais suffisamment rigide de façon à maintenir un passage artificiel dans cette dernière, comprend deux éléments tubulaires (8,9), dits respectivement supérieur (8) et inférieur (9), destinés à être placés respectivement dans la partie supérieure (10) et la partie inférieure (11 ) du canal urétral, de part et d'autre du sphincter strié (13), lesdits éléments tubulaires (8,9) étant reliés l'un à l'autre par un moyen de liaison (14) flexible et déformable, se logeant dans l'orifice (13a) du sphincter strié (13) ;
- le moyen d'introduction (3) comprend un tube poussoir (7), et une tige interne (19) de section extérieure adaptée à la section intérieure du tube externe (7), ledit moyen d'introduction ayant une longueur adaptée pour intuber le moyen de cathétérisation jusqu'à l'extrémité distale de l'élément supérieur (8)
**caractérisé en ce que,** le moyen d'introduction (3) comprend un moyen distal de positionnement provisoire (16), par rapport au coté inférieur du sphincter strié, et d'autre part le moyen d'introduction (3) comprend un moyen (29) extérieur d'activation du moyen de positionnement provisoire (16), disposé à l'extrémité proximale (25) du moyen d'introduction (3), et **en ce que**, premièrement le tube externe poussoir (7) a un diamètre extérieur moins important que le diamètre intérieur des deux éléments tubulaires (8, 9) du moyen de cathétérisation (2), de façon à permettre un déplacement longitudinal avec jeu fonctionnel du moyen de cathétérisation (2) sur le moyen d'introduction (3), deuxièmement le tube externe poussoir (7) a une longueur sensiblement égale à celle de la tige interne (19), et troisièmement le moyen de cathétérisation (2) est disposé sur le moyen d'introduction (3), en sorte que le moyen distal (16) de positionnement provisoire se situe entre les deux éléments tubulaires (8, 9) du moyen de cathétérisation (2).

2. Cathéter (1) selon la revendication 1, **caractérisé en ce que** le moyen distal (16) de positionnement provisoire comprend une pluralité d'éléments d'appui (51) distribués autour du tube externe poussoir (7), agencés chacun pour prendre deux positions, à savoir une position rétractée radialement, et une position déployée saillant radialement à partir du tube externe poussoir (7).

3. Cathéter (1) selon les revendications 1 et 2, **caractérisé en ce que**, premièrement la tige interne (19) a une longueur sensiblement égale à celle du tube externe poussoir (7), et est fixée en translation à son extrémité distale (19a) sur l'extrémité distale (7a) du tube externe poussoir (7), deuxièmement le tube externe (7) comporte localement des découpes axiales (50) ménageant entre elles des languettes (51), chacune pliable radialement entre deux positions, à savoir une position repliée en son milieu vers l'extérieur, et une position dépliée, se confondant dans la paroi du tube externe poussoir (7), lesdites languettes constituant ainsi lesdits éléments d'appui du moyen distal (16) de positionnement provisoire, et troisièmement le moyen extérieur (29) d'activation comprend un moyen pour déplacer axialement le tube externe (7) par rapport à la tige interne (19), et plier et déplier en correspondance les languettes (51) du moyen distal (16) de positionnement provisoire.

## Patentansprüche

1. Teilbarer Katheter (1) zur Verwendung bei der Behandlung von prostatischen Hindernissen beim Mann, mit einem distalen Katheterisierungsmittel (2) und einem Einführmittel (3), die zum Einführen des Katheters in die Harnröhre in miteinander fluchtender Ausrichtung zusammengefügt sind und voneinander trennbar sind, um das Katheterisierungsmittel (2) in der Harnröhre an Ort und Stelle zu belassen, wobei bei dem Katheter:
- das Katheterisierungsmittel (2), das relativ flexibel ist, so dass es sich an die Harnröhre des Prostatabereiches anpassen kann, jedoch ausreichend steif ist, um einen künstlichen Durchgang in Letzterer aufrecht zu erhalten, zwei rohrförmige als oberes (8) bzw. unteres (9) bezeichnete rohrförmige Elemente (8, 9) aufweist, die dazu bestimmt sind, in der oberen Partie (10) bzw. der unteren Partie (11) des Harnröhrenkanals beidseits des Musculus sphincter (13) angeordnet zu werden, wobei die rohrförmigen Elemente (8, 9) miteinander durch ein flexibles und verformbares Verbindungsmittel (14) verbunden sind, das sich in der Öffnung (13a) des Musculus sphincter (13) platziert;
- das Einführmittel (3) ein Schubrohr (7) und einen inneren Schaft (19) mit einem Außenquerschnitt aufweist, der auf den Innenquerschnitt des äußeren Rohres (7) ausgelegt ist, wobei das Einführmittel eine Länge aufweist, die dazu ausgelegt ist, das Katheterisierungsmittel bis zum distalen Ende des oberen Elements (8) zu intubieren;
**dadurch gekennzeichnet, dass** das Einführmittel (3) ein distales Positioniermittel (16) zur vorläufigen Positionierung bezüglich der unteren Seite des Musculus sphincter aufweist, und andererseits das Einführmittel (3) ein äußeres Aktivierungsmittel (29) zur Aktivierung des Positioniermittels (16) zur vorläufigen Positionierung aufweist, das am proximalen Ende (25) des Einführmittels (3) angeordnet ist, und dass erstens das äußere Schubrohr (7) einen Außendurchmesser aufweist, der geringer als der Innendurchmesser der beiden rohrförmigen Elemente (8, 9) des Katheterisierungsmittels (2) ist, derart, dass eine längs gerichtete Verschiebung mit einem Funktionsspiel des Katheterisierungsmittels (2) auf dem Einführmittel (3) ermöglicht wird, zweitens das äußere Schubrohr (7) eine Länge aufweist, die etwa gleich derjenigen des inneren Schaftes (19) ist, und drittens das Katheterisierungsmittel (2) auf dem Einführmittel (3) angeordnet ist, derart, dass das distale Positionierungsmittel (16) zur vorläufigen Positionierung sich zwischen den beiden rohr-förmigen Elementen (8, 9) des Katheterisierungsmittels (2) befindet.

2. Katheter (1) nach Anspruch 1, **dadurch gekennzeichnet, dass** das distale Positionierungsmittel (16) zur vorläufigen Positionierung eine Mehrzahl von Anlageelementen (51) aufweist, die um das äußere Schubrohr (7) herum verteilt sind, und die jeweils dazu ausgestaltet sind, zwei Positionen einzunehmen, nämlich eine radial zurückgezogene Position und eine von dem äußeren Schubrohr (7) radial vorspringende ausgefahrene Position.

3. Katheter (1) nach den Ansprüchen 1 und 2, **dadurch gekennzeichnet, dass** erstens der innere Schaft (19) eine Länge aufweist, die etwa gleich derjenigen des äußeren Schubrohrs (7) ist, und bezüglich einer translatorischen Bewegung an seinem distalen Ende (19a) an dem distalen Ende (7a) des äußeren Schubrohrs (7) festgelegt ist, zweitens das äußere Rohr (7) lokal axiale Schnitte (50) aufweist, die zwischen sich Laschen (51) ausbilden, die jeweils radial zwischen zwei Positionen faltbar sind, nämlich einer in ihrer Mitte nach außen zusammengefalteten Position und einer entfalteten Position, in der sie sich in die Wand des äußeren Schubrohrs (7) einfügen, wobei die Laschen so die Anlagemittel des distalen Positionierungsmittels (16) zur vorläufigen Positionierung bilden, und drittens das äußere Aktivierungsmittel (29) ein Mittel aufweist, um das äußere Rohr (7) bezüglich des inneren Schafts (19) axial zu verschieben und entsprechend die Laschen (51) des distalen Positionierungsmittels (16) zur vorläufigen Positionierung zu falten und zu entfalten.

## Claims

1. Separable catheter (1) intended to be used in the treatment of prostate obstructions in man, comprising a distal means of catheterization (2) and a means of introduction (3) which are joined to one another in an aligned manner, for introducing the catheter into the urethra, and which can be disconnected from one another, for leaving the catheterization means (2) in place in the urethra, in accordance with which catheter:
- the catheterization means (2), which is relatively flexible so as to conform to the urethra in the prostate region, but sufficiently rigid to maintain an artificial passage in the latter, comprises two tubular elements (8, 9), termed upper (8) and lower (9) respectively, which are intended to be placed in the upper part (10) and lower part (11), respectively, of the urethral channel, on either side of the striated muscular sphincter (13), said tubular elements (8, 9) being connected to one another by a flexible and deformable connection means (14) which lodges itself in the orifice (13a) of the striated muscular sphincter (13);
- the introduction means (3) comprises a pusher tube (7), and an inner rod (19) whose external cross section is adapted to the internal cross section of the outer tube (7), said introduction means having a length adapted in order to intubate the catheterization means as far as the distal end of the upper element (8),
**characterized in that** the introduction means (3) comprises a distal means (16) for temporary positioning in relation to the lower side of the striated muscular sphincter, and, moreover, the introduction means (3) comprises an outer means (29), for actuation of the temporary positioning means (16), arranged at the proximal end (25) of the introduction means (3), and **in that**, firstly, the outer pusher tube (7) has an external diameter smaller than the internal diameter of the two tubular elements (8, 9) of the catheterization means (2) , so as to permit a longitudinal displacement, with functional play, of the catheterization means (2) on the introduction means (3), secondly, the outer pusher tube (7) has a length substantially equal to that of the inner rod (19), and, thirdly, the catheterization means (2) is arranged on the introduction means (3) in such a way that the distal temporary positioning means (16) is situated between the two tubular elements (8, 9) of the catheterization means (2).

2. Catheter (1) according to Claim 1, **characterized in that** the distal temporary positioning means (16) comprises a plurality of bearing elements (51) which are distributed about the outer pusher tube (7) and are each designed to assume two positions, namely a radially retracted position, and a deployed position protruding radially from the outer pusher tube (7).

3. Catheter (1) according to Claims 1 and 2, **characterized in that**, firstly, the inner rod (19) has a length substantially equal to that of the outer pusher tube (7) and is fixed in terms of translation at its distal end (19a) on the distal end (7a) of the outer pusher tube (7), and, secondly, the outer tube (7) includes axial cutouts (50) locally which form between them tongues (51), each radially foldable between two positions, namely a position folded outward at the centre, and an unfolded position, merging in the wall of the outer pusher tube (7), said tongues thus constituting said bearing elements of the distal temporary positioning means (16), and, thirdly, the outer actuation means (29) comprises a means for axially displacing the outer tube (7) in relation to the inner rod (19) and for correspondingly folding and unfolding the tongues (51) of the distal temporary positioning means (16).
